# EUROPEAN PATENT SPECIFICATION

(11) **EP 2 050 393 B1**
(45) Date of publication and mention of the grant of the patent: **13.04.2011**
(21) Application number: 07118860.1
(22) Date of filing: 19.10.2007
(51) Int. Cl.: A61B 5/151

(54) **Lancing device**
Stechvorrichtung
Dispositif de lancement

(43) Date of publication of application: 22.04.2009
(73) Proprietor: Bionime Corporation, Taichung County (TW)
(72) Inventor: Michel, Thomas, 8006 Zürich (CH); Larsson, Marita, 9000 St. Gallen (CH); Huang, Mao-Sung, Dali City, Taichung County 412 (TW); Huang, Kuang-Li, Dali City, Taichung County 412 (TW)
(74) Representative: Hepp, Dieter

(56) References cited:
- EP-A- 0 569 124
- EP-A- 1 614 383
- DE-B4- 19 781 098
- US-A- 4 503 856
- US-A1- 2005 090 850

## Description

The present invention relates to a lancing device for ejecting a lancet according to the preamble of the independent claim.

Many medical procedures in use today require a relatively small sample of blood, in the range of 5-50 µl. Normally such a sample is obtained by lancing or piercing the skin at a selected location, such as the finger, to enable the collection of 1 or 2 drops of blood. With the advent of home use tests such as self monitoring of blood glucose, there is a requirement for a simple and safe procedure which can be performed by a person needing to test.

Lancets in conventional use generally have a rigid body and a sterile pricking element, for example a needle, which protrudes from one end. The lancet may be used to pierce the skin, thereby enabling the collection of a blood sample from the opening created. The blood is transferred to a test device or collection device. Blood is most commonly taken from the fingertips, where the supply is generally excellent. However, blood can also be taken of alternate sites, such as earlobes and limbs.

To reduce the anxiety of piercing the skin and to guarantee a reproducible penetration result many spring loaded lancing devices have been developed.

The lancing device according to the present invention is defined in claim 1 and comprises a lancet holder, which is slideable in a housing member between a cocked position and a pricking position. In use the lancet holder carries a lancet which comprises a pricking element protruding there from along an ejection axis. The lancing device further comprises a front cap, which has an opening against which the object to be pricked is to be placed. The pricking element extends through the opening when the lancet is ejected.

Typically the lancet holder is axially slideable along the main axis of a generally tubular housing in forward direction into the pricking position and in backward direction for being loaded.

Conventional lancing devices, as for example disclosed in EP 0 115 388, typically require a user to put a lancet in a socket of a lancing device, arm the lancing device by latching the lancet holder in a retracted position, urge the lancing device against a target site, and then press a release button or other switch to manually activate the lancing device such that a lancet within the device is launched (also referred to as "fired") towards the target site. The lancet then penetrates the target site, thereby creating an opening for the expression of a bodily fluid sample.

After the lancet pierces the skin, a bounce back spring retracts the lancet to a safe position within the device. Now the lancet can be removed from the lancing device.

Typical lancing devices have a removable front cap, preferably also suited for adjusting the penetration depth. Once the front cap is removed the used lancet can be taken out of a socket of the lancet holder and a new lancet can be put in. When the front cap is removed from the body of the lancing device, the pricking element is exposed and there is a certain risk of injury and of contamination for the person who removes or changes the lancet. Known lancing devices generally are not protected from being fired unintentionally.

US 4,503,856 describes a lancing device having a lancet holder and a spring disposed in a housing. An external control member is connected to the holder to move the holder to a retracted position against the force of a spring. The control member is movable to release the holder to allow the holder to move to a lancet skin piercing position and then to a withdrawn position.

EP 1 614 383 discloses a lancing device with an ejection mechanism for used lancets. A lancet is inserted in a lancet holder which is movable in a lancing direction and a retreating direction. Ejection of the lancet is done by pushing the ejection switch in the lancing direction, whereby a pin will subsequently push the lancet outward.

Even though many improvements have been made, the handling of the known lancing devices is still complicated for the user and there still is a risk of injury and contamination while replacing used lancets. It is the object of the present invention to overcome the drawbacks of the known devices, in particular to facilitate the change of the lancet, to prevent unintentional lancing and to provide a safe handling, especially of a lancing device with a used lancet.

According to a first aspect of the invention the lancing device possesses a safety switch. Said safety switch is moveable between a safety position, in which it prevents the lancet holder from being cocked and a passing position, in which it does not prevent cocking and/or movement of the lancet holder.

When the safety switch is in the safety position and thus the lancet holder is prevented from being cocked, the lancet cannot be fired from a cocked position with a high speed. By operating the safety switch the user can control the ability of the lancing device of firing the lancet.

The safety switch can for example be brought into the safety position, when the lancet was fired once and shall be changed. The risk for the person changing the lancet of being hit accidentally by the lancet is thereby reduced. Furthermore in a state, where the housing member is open to change the lancet, the lancet cannot be ejected by an unintended firing.

The safety switch may prevent the lancet holder from being cocked for example by blocking the movement of the lancet holder into the cocked position or by preventing latching of a clutch member on the lancet holder in a corresponding member of the housing.

When any movement of the lancet holder is blocked in the safety position a person may easily remove a lancet from or place a lancet into the socket of the lancet holder. The positioning of the lancet relatively to the lancet holder is facilitated, when the lancet holder does not move. The top of a used lancet can easily be stuck into a protective cover, if the lancet holder does not back off.

According to a second aspect of the invention the lancing device comprises an ejection pin for pushing the lancet out of the lancet holder, in combination with the safety switch as described above. The ejection pin is moveable with respect to the lancet holder. The lancing device further comprises an ejection switch which is moveable between an ejection position, in which it allows relative movement of the ejection pin and the lancet holder, and a loading position, in which it does not affect a coupled movement of the ejection pin and the lancet holder.

For ejection of the lancet the ejection pin is used to push the lancet out of the socket of the lancet holder, thus there is no need for a person touching the lancet after use, especially the part of the lancet where the pricking element or needle protrudes. Preferably the ejection pin is arranged at the lancet holder and can protrude into the socket when the lancet shall be pushed out.

According to this aspect of the invention the lancing device comprises an ejection switch with two positions. In the first position, the so called ejection position, the ejection pin can be moved relatively to the lancet holder and the lancet can be pulled off the socket of the lancet holder.

The ejection switch may also take a second position, the so called loading position, in which the movement of the ejection pin and the lancet holder are coupled. In this position a lancet generally rests in the socket of the lancet holder.

In a preferred embodiment the ejection switch can be set in a loading position, in which it does not affect the movement of the ejection pin, especially along with a backward movement of the lancet holder.

In the ejection position the ejection switch provides a blocking of the movement of the ejection pin with respect to the housing of the lancing device. When the lancet holder is pulled backward, it is moved with respect to the ejection pin, which rests in its forward position and pushes the lancet out of the socket.

If the ejection switch is in the loading position and the lancet holder is pulled back in this position, the ejection pin moves along with the lancet holder and a lancet, being held in the socket, is also retracted. Thus the lancet holder can be pulled in the cocked position while carrying a lancet and the lancing device is loaded and ready to fire.

The backward movement of the lancet holder can both be used for loading the lancing device and for ejecting a used lancet.

The safety switch and the ejection switch are mounted on a switch member. The safety switch and the ejection switch are mounted on the same switch member.

Since the safety and the ejection position are especially suitable for changing the lancet the safety switch and the ejection switch are then simultaneously either in the safety and ejection position or in the passing and loading position.

Preferably the switch member is arranged between an outer and an inner housing. On the one hand the switch member can be moved independently of the position of the lancet holder and on the other hand the switch member may affect elements of the inner housing to contact elements of the lancet holder. More preferably the switch member is a tubular element.

In an advantageous embodiment the switch member is axially slideable inside the housing, preferably the outer housing. Thus the switch can be in a first position, in which it is preferably placed in its most forward position and in a second position, in which it is preferably in its most backward position.

In a further advantageous embodiment the lancing device comprises a safety spring for pushing the switch member, preferably into the ejection direction. Thus the switch member has a preferred position and the safety switch and/or the ejection switch also have a preferred switch position.

In a preferred embodiment the front cap is removable. The safety switch is brought into the safety position and/or the ejection switch is brought into the ejection position when the front cap is removed. Thus as soon as the lancing device is opened, for example for taking a used lancet or placing a new lancet, the safety switch and/or the ejection switch take the position , which is appropriate for changing the lancet.

In an advantageous embodiment the safety switch is held back in the passing position by the front cap and/or the ejection switch is held back in the loading position by the front cap, preferably against the spring force of a safety spring, as long as the front cap is attached. Thus the safety and/or ejection switch is operated by the removeable cap. Since the user anyway attaches the front cap after changing the lancet the safety and/or ejection switch is brought into the appropriate state automatically without any obligation for the user to separately operate the safety and/or ejection switch. Preferably the cap has a stopping face which presses the switch member is backward direction as long as the cap is attached. When the cap and thus the stopping face are removed the safety spring may actuate the switch member in ejection direction. The switch member can take the most forward position and the safety switch is in the safety position and/or the ejection switch is in the ejection position.

The lancing device according to the present invention may further comprise an operating means, especially a means for releasing the lancet holder from the cocked position. The lancing device is designed is such a way that when the safety switch is in the safety position it prevents the movement of the lancet holder towards the cocked position depending on the operation of the operating means, in particular as long as the operating means is not operated.

Alternatively the lancet holder may rest cocked in the forward position and is not moveable in the backward direction until the operating means are operated.

The means for releasing the lancet holder from the cocked position may be a push button arranged on the outside of a tubular body of the lancing device. Alternatively it may be a push button on one of the ends of the lancing device, it may also be turning knob, a lever, a touch field or any other operating element.

The means for releasing the lancet holder from the cocked position therefore not only may release the lancet holder from the cocked position. It also may allow the movement of the lancet holder towards the cocked position even if the safety switch is in the safety position. The lancet holder may preferably be moved in a backward position when the lancet has been fired once and is used. A backward movement of the lancet holder without achieving the cocked position may be useful for ejecting the lancet, when for example the ejection switch prevents the backward movement of the ejection pin along with the lancet holder.

In a preferred embodiment of the invention the front cap has to be removed for changing the lancet. When the cap is removed of the lancing device after firing the lancet, the switch member is pressed in ejection direction by a safety spring and brings the safety switch into the safety position and the ejection switch into the ejection position.

Once the cap is removed and the safety switch is in safety position, the lancet holder cannot be moved backwards into the cocked position. Thus there is no uncontrolled movement of the lancet bearing the risk of a sudden and accidental contact between the pricking element and the user. If the user wants to take off the lancet there is no need of touching the lancet. The user only presses the release button to allow a backward movement of the lancet holder and retracts a loading means coupled with the lancet holder in the same way he would do if he wants to load the lancing device. Thereby the user pulls the lancet holder backwards in the direction of the cocked position.

The safety switch still is in the safety position prevents the lancet holder from being cocked, for example by preventing a clutch element of the lancet holder to be latched at a locking face of the housing member. At the same time the ejection switch still is in the ejection position and prevents the ejection pin from being moved together with the lancet holder. Thus when the lancet holder is pulled back the ejection pin rests in its forward position and pushes the lancet out of the socket of the lancet holder.

Afterwards the user may let off the loading means and the release means. The lancet holder comes back to the front of the lancing device and is still prevented from being moveable in backward direction by the safety switch as long as the front cap is open. The user can put a new lancet into the socket of the lancet holder, the lancet holder not backing off when the lancet is pressed into the socket.

When the front cap is attached, the switch means again is brought into backward position. The safety switch thereby is brought into the passing position and the ejection pin is brought into the loading position. The lancing device is ready to be loaded and to be fired.

In an advantageous embodiment of the invention the lancing device comprises an outer housing and an inner housing. The inner housing comprises a first cantilever beam for engagement with a stopping face of a clutch element of the lancet holder and/or the inner housing comprises a second cantilever beam for engagement with a stopping face of the ejection pin. Thus the safety switch and/or the ejection switch may affect elements of the lancet holder via a cantilever beam of the inner housing. The position of the safety switch and/or of the ejection switch, especially when arranged on a safety switch, is completely independent from the position of the lancet holder and of the movement of the lancet holder.

Preferably the switch member is arranged between the outer housing and the inner housing. The switch member and thereby the safety switch and/or the ejection switch are shielded by the outer housing, and thus for example protected from being soiled or collecting dust, what could have a negative effect on the movement of the switch element.

According to a preferred aspect of the invention the lancing device comprises a loading member for moving the lancet holder in the cocked position and an ejection pin for pushing the lancet out of the lancet holder. The ejection pin is moveable with respect to the lancet holder. The lancing device further comprises a mode switch member for switching between a using state, wherein operating the loading member provides a cocking of the lancet holder, and an exchange state wherein operating the loading member provides an ejection of a lancet off the lancet holder.

The loading member of the lacing device on the one hand can be used to bring the lancing device in a ready-to-fire state, when the switch member is in the using state, on the other hand the same loading member serves as an ejection tool. The lacing device therefore has no unnecessary operating elements and can be constructed in a compact and cost-efficient way. There is no need for separate security elements preventing unintended operation, when the lancing device is not in the appropriate state.

More preferably the mode switch member is a switch member and comprises a safety switch and an ejection switch as described above.

In a preferred embodiment of the lancing device with a mode switch member the lancing device further comprises a removable front cap. The lacing device is construed such that the switch member is brought into the using state, when the front cap is attached and the mode switch member is brought into the exchange state, when the front cap is removed.

The switch member thus is operated automatically by placing the front cap.

According to a further preferred aspect of the invention the lancing device, preferably as described above, comprises an outer housing and an inner housing, the inner housing comprises at least two guide tracks, which guide at least two corresponding guide ribs being arranged on the lancet holder.

When the lancet held in the lancet holder is propelled in the puncture direction, it should perform a stable and smooth movement at high velocity along a predetermined puncturing path to reduce the pain. The guiding of the guide ribs in the guide tracks prevents rotational components of the movement and reduces vibrations during forward movement.

Preferably the inner housing comprises four guide tracks, which guide four corresponding guide ribs in the lancet holder. The guidance if the lancet holder is stabilized in perpendicular lateral directions and the resulting lateral tolerances are much smaller compared with a cylindrical guidance. A rectilinear advancement of the lancet holder reduces the pain during the needle insertion.

Further benefits and advantages of the present invention will be described in the description and with reference to the following drawings.

### Brief description of the drawings:

- Figure 1: is a perspective view of a lancing device;
- Figure 2: is a cross sectional side view of the lancing device in Figure 1, along a plane AA, with a safety switch in a passing position;
- Figure 3: is a cross sectional side view of the lancing device in Figure 1, along a plane CC, with an ejection switch in loading position;
- Figure 4: is a cross sectional side view of the lancing device in Figure 1, along a plane AA, without front cap and with the safety switch in a safety position;
- Figure 5: is a cross sectional side view of the lancing device in Figure 1, along a plane CC without front cap and with the ejection switch in ejecting position;
- Figure 6: is another cross sectional side view of the lancing device in Figure 1, along a plane AA without front cap and with a loading member being pulled backwards;
- Figure 7: is a perspective view of the lancing device without front cap;
- Figure 8: is a perspective view of a lancet holder;
- Figure 9: is a perspective view of a lancet holder and an ejection pin;
- Figure 10: is a side view of a lancet holder and an ejection pin;
- Figure 11: is a side view of a lancet holder with an ejection pin;
- Figure 12: is an exploded view on all parts of the lancing device.

The embodiments described below are merely exemplary and are not intended to limit the invention to the precise forms disclosed.

Instead, the embodiments were selected for description to enable one of ordinary skill in the art to practice the invention.

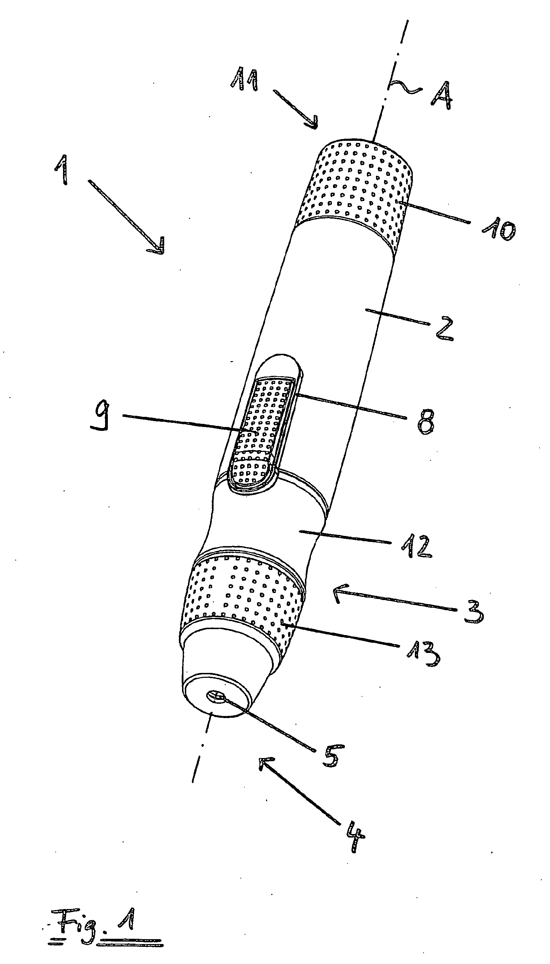

FIG. 1 shows a perspective view of a lancing device 1. The lancing device 1 comprises an elongated outer housing 2 extending along the main axis A of the device 1. A removable cap 3 is disposed at the front end 4 of the housing 2 and has an exit opening 5 for the pricking tip 6 of a lancet 7, not shown in the figure.

The housing includes an orifice 8 which provides access to a release button 9 that is used to fire a lancet 7. A loading member 10 extends from the backward end 11 of the housing 2 and is used to arm the lancing device 1 prior to firing the lancet 7 with the release button 9.

The removable cap 3 comprises a cap holder 12 and a revolvable outer cap 13, by which the penetration depth can be adjusted in a manner known to those skilled in the art.

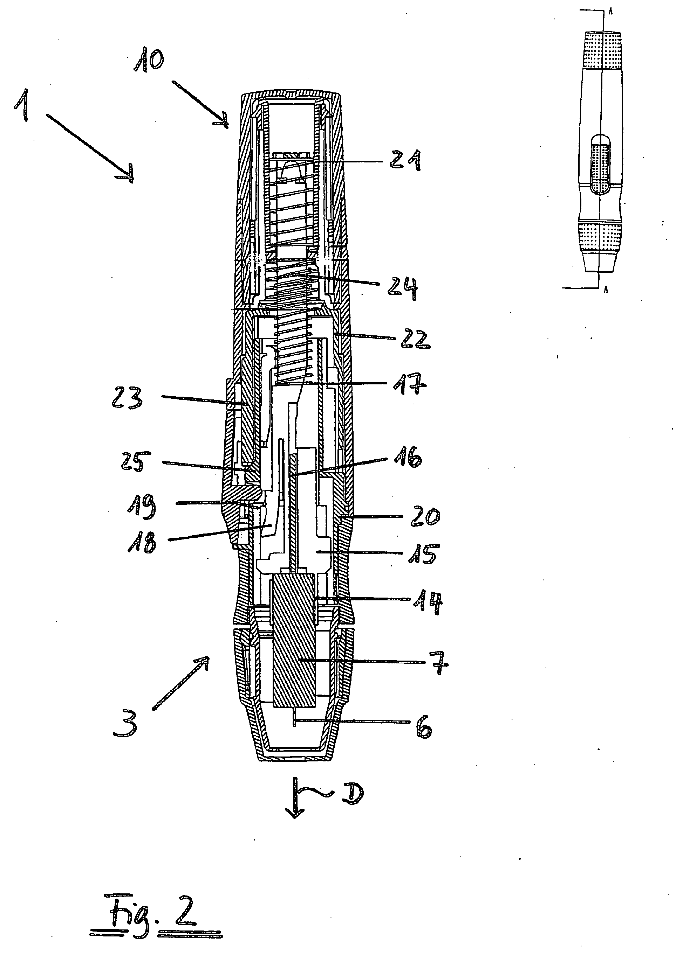

Figure 2 shows a cross sectional side view of lancing device 1 along a plane AA. A lancet 7 is mounted in a socket 14 of a lancet holder 15. The lancet holder 15 is movable in a lancing direction D between a retracted position in which the lancet tip 6 does not extend outside of the end cap 3 and an extended position in which the lancet 7 is adapted to make a puncture having a certain puncture depth.

The lancet 7 is held by the socket 14 of the lancet holder 15 in such a fashion that the position of the tip 6 relative to the holder 15 is reproducibly the same when a new lancet 7 is inserted into the holder for subsequent blood withdrawal. The lancet holder 15 comprises an ejection pin 16, which has a position out of the socket 14 when a lancet 7 is positioned in the socket 14.

The pricking action is driven by a fire spring 17, which is loaded by pulling back the loading member 10 in opposition to its spring force.

After loading, the clutch element 18 of the lancet holder is locked in the cocked position using a locking face 19 on the inner housing 20 (not shown in detail in FIG. 2). The loading member 10 returns to the original position as shown in the figure.

An opening 5 of a front cap 3 is pressed against a body part in which a pricking is to be achieved in order to obtain a drop of blood. After pressing the release button 9 the lancet holder 15 then makes a puncturing motion to propel the lancet 7, held in the lancet holder 15, at high velocity along a predetermined puncturing path preferentially extending along the main axis A in the puncture direction D until its tip 6 exits from the opening 5 and penetrates into the body part. The lancet 7 then returns to its initial position inside the lancing device 1.

The return motion of the lancet holder 15, and thereby the lancet 7, as well as of the loading member 10 is driven by a return spring 21.

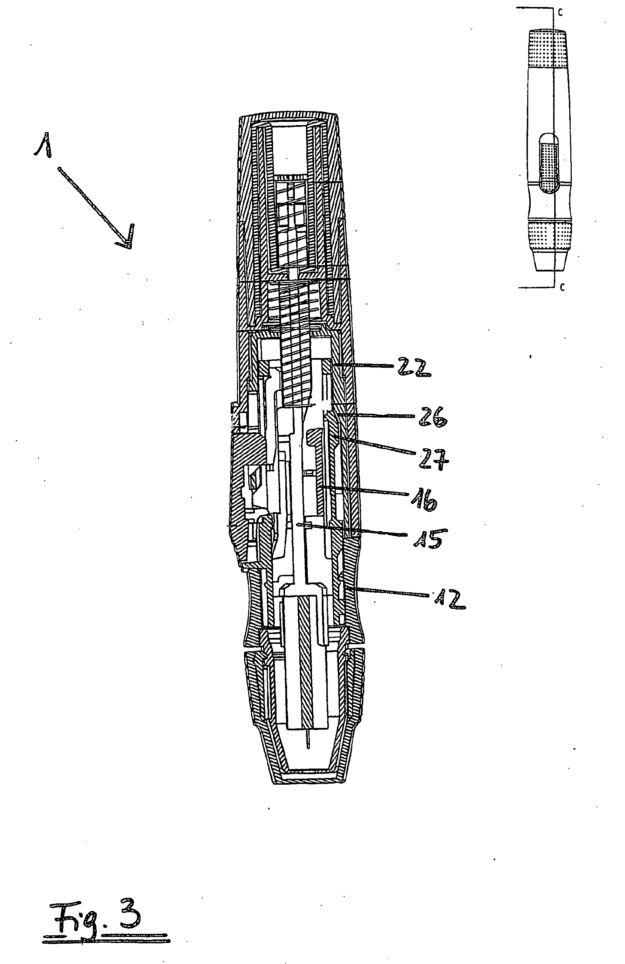

The lancing device 1 further comprises a switch member 22 with a safety switch 23. The switch member is held in backward position by the cap holder 12 against the spring force of a safety spring 24 (as may be better seen in Figure 3). Since in this position the safety switch 23 does not prevent cocking and/or movement of the lancet holder it is in a so called "passing position".

The switch member is positioned between the inner housing 20 and the outer housing 2, such that the safety switch may affect a first cantilever beam 25 of the inner housing 20.

The lancet device 1 as shown in figure 2 is ready to be loaded by pulling the back bar 10, thus the switch member 22 is in the so called "using state".

As presented in Figure 3, which shows a cross sectional side view of a lancing device 1 along an axis CC, the switch member 22 also comprises an ejection switch 26 which may affect a second cantilever beam 27 of the inner housing 20.

In the position shown in the figure the ejection switch 26 does not affect a coupled movement of the ejection pin 16 and the lancet holder 15, which is necessary for avoiding the ejection of the lancet 7 off the lancet holder 15 and thus for loading the lancing device 1, thus the ejection switch 26 is the so called "loading position".

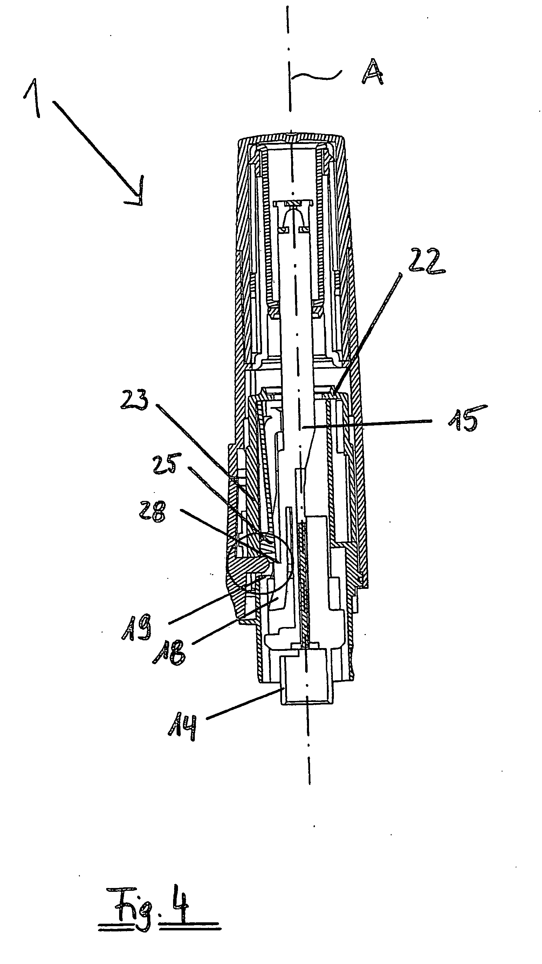

Figure 4 presents a cross sectional side view of the lancing device 1, along a plane AA, without front cap 3 and with the safety switch 23 in safety position. When the front cap 3 is removed, the switch member 22 is urged in pricking direction D by the safety spring 24 (not shown in the figure). The safety switch 23 thereby affects the first cantilever beam 25 which is inclined towards the main axis A and engages a stopping face 28 of a clutch element 18 of the lancet holder 15. In this state the lancet holder 15 cannot back off against the pricking direction D.

If a new lancet (not shown in Figure 4) shall be put into the socket 14 of the lancet holder 15 the lancet holder 15 is restricted from backward movement, what makes the charging of the lancing device 1 comfortable and secure for a user.

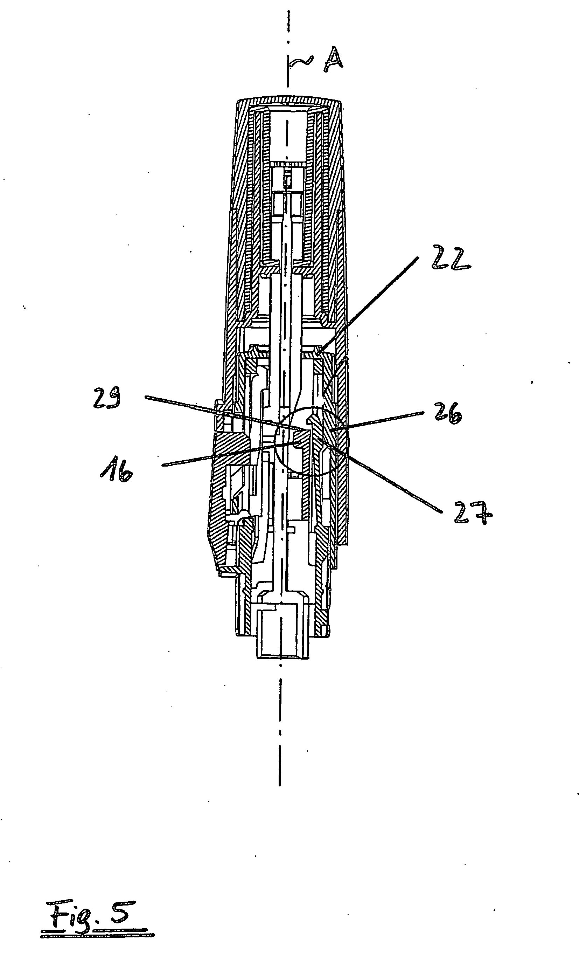

Figure 5 presents a cross sectional side view of the lancing device 1, along a plane CC, without front cap 3.

As long as the front cap 3 remains removed, the switch member 22 is urged in pricking direction D. The ejection switch 26 thereby affects the second cantilever beam 27 which is also inclined towards the main axis A and which engages a stopping face 29 of the ejection pin 16 in a hooking manner. The ejection pin 16 is prevented from being moved along with the lancet holder 15, the ejection switch 26 thus is in the so called "ejection position".

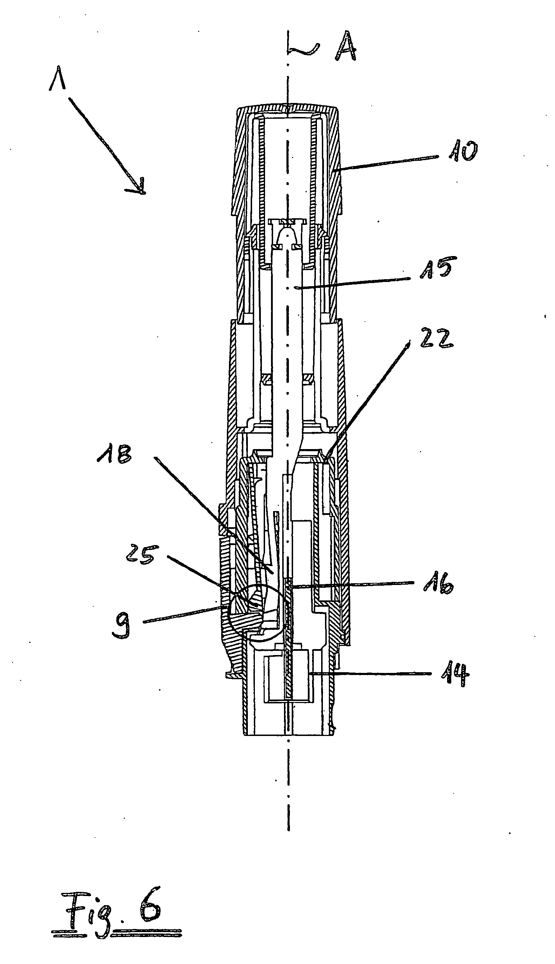

As can be seen in Figure 6, showing another cross sectional view of the lancing device 1, when the cap 3 is removed and the release button 9 is pressed, the clutch element 18 of the lancet holder 15 is urged towards the main axis A of the lancing device 1 and may pass the first cantilever beam 25, when the back bar 10 is pulled. Thus the lancet holder 15 can be retracted backwards.

In this position the safety switch 23 still prevents the lancet holder 15 from being cocked, because it does not allow the clutch element 18 to be hold by the locking face 19 in the inner housing 20, it is thus in the so called "safety position". Though the lancet holder 15 can be moved, it cannot be cocked.

Since the switch member 22 still is in forward position, the ejection switch still affects the second cantilever beam 27, which engages the ejection pin 16 (not shown in the figure). The ejection pin 16 thereby is prevented from being moved along with lancet holder 15. The ejection pin 16 rests in its forward position and penetrates the socket 14 of the lancet holder 15.

When a lancet 7 (not shown in the figure) is held in the socket 14 it is ejected by the ejection pin 16.

If the switch element 22 is in the forward position, pulling the loading member 10 does not result in loading the lancing device, because the lancet holder 15 cannot been cocked, but rather in ejecting a lancet from the lancet holder 15, the switch element 22 corresponds to a mode switch element 22 which is in the so called "exchange state".

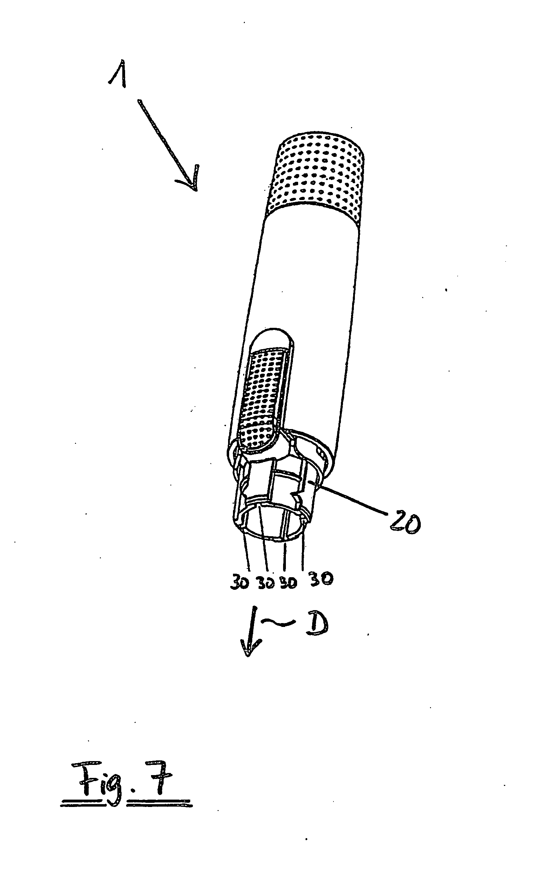

Figure 7 shows a perspective view of a lancing device 1 without front cap 3. On the inside of the inner housing 20 there are four guide tracks 30 for accommodating guide ribs 31 of the lancet holder 15.

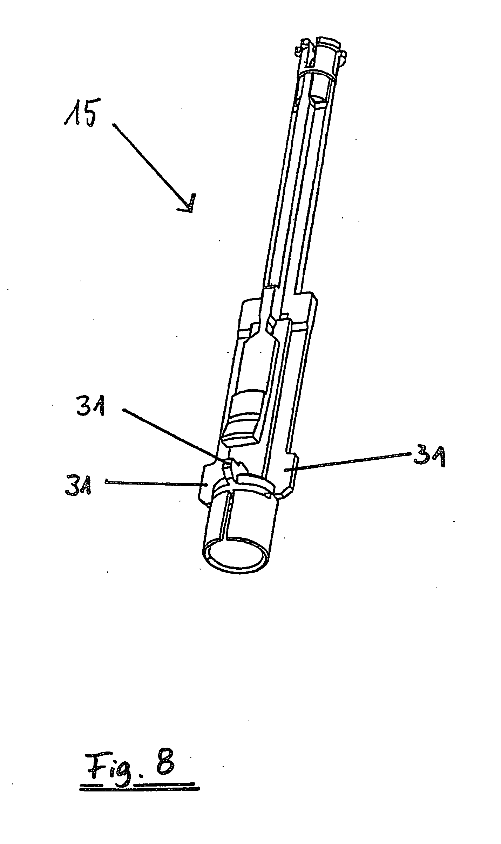

Figure 8 shows a perspective view of a lancet holder 15 with four guide ribs. The guiding of the guide ribs 31 in the guide tracks 30 guarantees a stable and smooth movement of the lancet 7, when the lancet holder is propelled in lancing direction D.

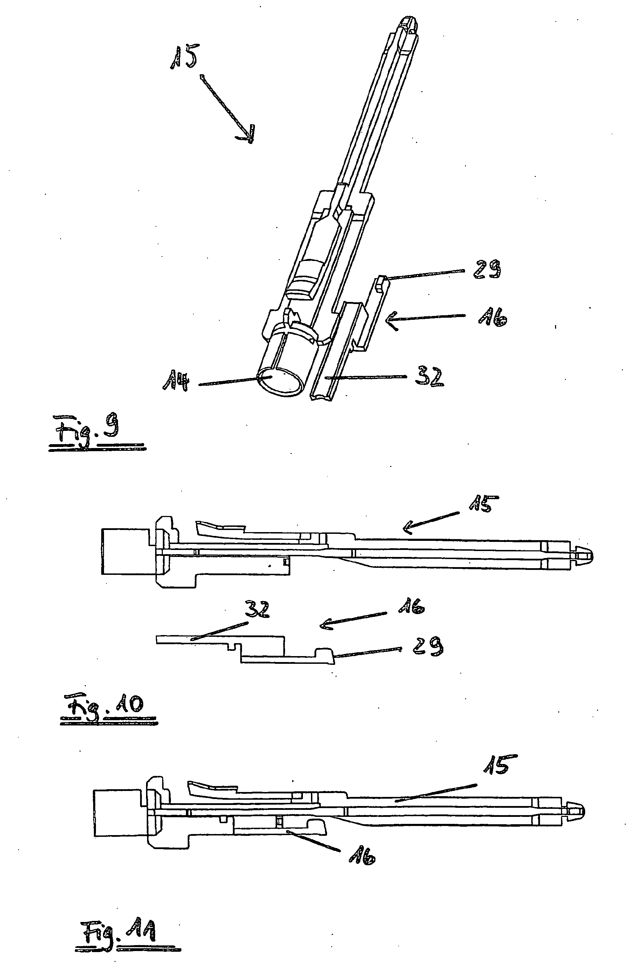

Figure 9 is a perspective view of the lancet holder 15 and the ejection pin 16, whereas figure 10 shows a side view of the lancet holder 15 and the ejection pin 16.

The ejection pin 16 has two parts. A first part 32 may protrude in the socket 14 to eject the lancet. A second part can be arranged on the outside of the socket 14 and comprises a stopping face for being hooked by the second cantilever beam 27 of the inner housing 20 not shown in the figure.

Figure 11 is a side view of a lancet holder 15 with an ejection pin 16. The ejection pin 16 is plugged in the lancet holder 15 and lancet holder 15 and ejection pin 16 are moveable relatively to each other, such that the first part 32 of the ejection pin 16 may shifted to the inside of the socket 14.

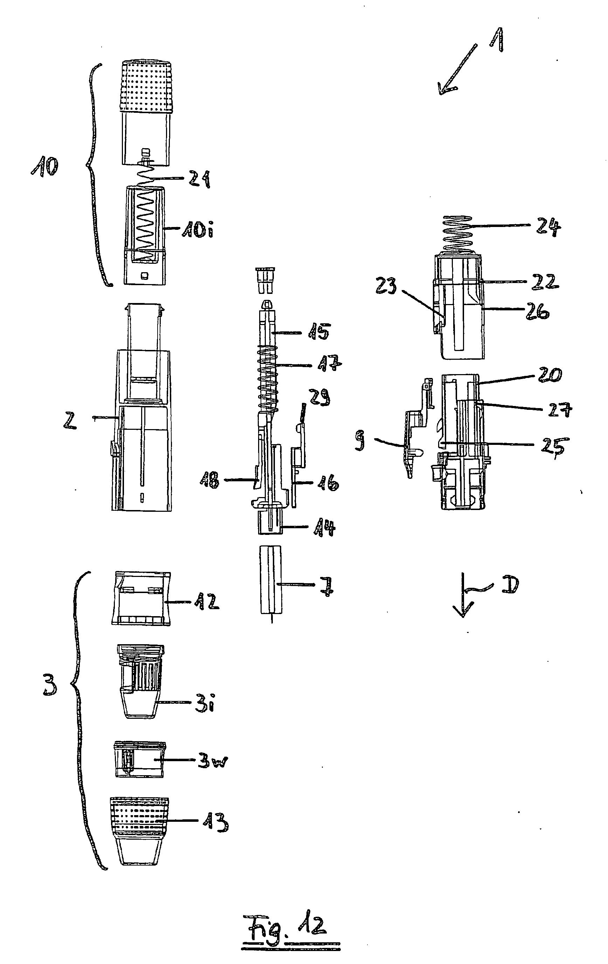

Figure 12 is an exploded view on all parts of the lancing device 1.

The outer body of the lancing device 1 consist of the outer housing 2, which accommodates a loading member 10, comprising an inner loading member 10i and a return spring 21. Fixable to the outer housing is a removable cap 3. The cap 3 is adapted to adjust the penetration depth and therefore comprises a preferable transparent turnable outer cap 13, a window cap 3w and an inner cap 3i.

The lancet 7 may be inserted into a socket 14 of a lancet holder 15, by which the lancet 7 may be activated when the fire spring 17 is loaded and released.

The lancet 7 may be ejected from the socket 14 by an ejection pin 16.

The clutch member 18 of the lancet holder 15 cooperates with parts of the inner housing 20. It may for example abut against a first cantilever beam 25 when the first cantilever beam 25 is pressed inwardly by the safety switch 23 of the safety member 22.

The safety member 22 further comprises an ejection switch 26, which may interact with a second cantilever beam 27 for holding the a stopping face 29 of the ejection pin 16.

The safety member 22 is actuated in lancing direction D by a safety spring 24.

## Claims

1. A lancing device for ejecting a lancet having a pricking element protruding there from along an ejection axis, comprising a front cap (3), having an opening (5) against which an object to be pricked can be placed, the pricking element extending through the opening (5) when the lancet is rejected, a lancet holder (15), slideable between a cocked position and a pricking position wherein
the lancing device (1) comprises
a safety switch (23), said safety switch (23) being moveable between a safety position, in which it prevents the lancet holder (15) from being cocked and a passing position, in which it does not prevent cocking and/or movement of the lancet holder (15), **characterized in that** it further comprises
an ejection switch (26), said ejection switch (26) being moveable between an ejection position, in which it allows relative movement of an ejection pin (16) and the lancet holder (15) for ejecting a lancet, and a loading position, in which it does not affect a coupled movement of the ejection pin (16) and the lancet holder (15),
whereby the safety switch (23) and the ejection switch (26) are mounted on a common switch member (22).

2. A lancing device according to claim 1, **characterized in that** said ejection switch (26) in the loading position affords the movement of the ejection pin (16), especially along with a backward movement of the lancet holder (15).

3. A lancing device according to claim 1, **characterized in that** the switch member (22) is axially slideable.

4. A lancing device according to claim 3, **characterized in that** lancing device (1) comprises a safety spring (24) for pushing the switch member (22).

5. A lancing device according to one of claims 1-4, **characterized in that** the front cap (3) is removable and the safety switch (23) is able to be brought in the safety position by removing the front cap (3) and/or the ejection switch (26) is able to be brought in the ejection position by removing the front cap (3).

6. A lancing device according to one of claims 1-5, **characterized in that** the safety switch (23) is held in the passing position by the front cap (3) and/or the ejection switch (26) is held in the loading position by the front cap (3), preferably against the spring force of a safety spring (24), as long as front cap (3) is attached.

7. A lancing device according to one of claims 1-6, **characterized in that** the lancing device (1) comprises an operating means (9), preferably a means (9) for releasing the lancet holder (15) from the cocked position, and the lancing device (1) is construed such that the safety switch (23) being in the safety position prevents the movement of the lancet holder (15) towards the cocked position depending on the operation of the operation means, especially as long as the operating means (9) is not operated.

8. A lancing device according to one of claims 1-7, **characterized in that** the lancing device comprises an outer housing (2) and in inner housing (20), the inner housing comprising a first cantilever beam (25) for engagement with a stopping face (28) of a clutch element (18) of the lancet holder (15) and/or a second cantilever beam (27) for engagement with a stopping face (29) of the ejection pin (16).

9. A lancing device according to one of claims 1-8 **characterized in that** the lancing device (1) comprises a mode switch member (22) for switching between a using state, wherein operating a loading member (10) provides a cocking of the lancet holder (15), and an exchange state wherein operating the loading member (10) provides an ejection of a lancet (7) placed in the lancet holder (15).

10. A lancing device according to claim 9, **characterized in that** the lancing device (1) comprises a removable front cap (3) and the lancing device (1) is construed such that the mode switch member (22) is able to be brought in the using state, when the front cap (3) is attached and the mode switch member (22) is able to be brought into the exchange state, when the front cap (3) is removed.

11. A lancing device according to one of claims 1-10, comprising la lancet holder (15), axially slideable between a cocked position and a pricking position, **characterized in that** the lancing device (1) comprises an outer housing (2) and in inner housing (20), the inner housing (20) comprising at least two guide tracks (30) which guide at least two corresponding guide ribs (31) on the lancet holder (15).

## Patentansprüche

1. Lanzettenvorrichtung zum Auswerfen einer Lanzette mit einem Stechelement, welches entlang eine Auswurfachse daraus hervorragt, umfassend: eine Vorderkappe (3) mit einer Öffnung (5), gegen die ein anzustechender Gegenstand angeordnet werden kann, wobei sich das Stechelement durch die Öffnung (5) hindurch erstreckt, wenn die Lanzette ausgeworfen wird, einen Lanzettenhalter (15), der zwischen einer gespannten Position und einer Stechposition hin und her gleiten kann, wobei die Lanzettenvorrichtung (1) einen Sicherheitsschalter (23) umfasst, der zwischen einer Sicherheitsstellung, in der er das Spannen des Lanzettenhalters (15) verhindert, und einer Passierstellung, in der er das Spannen und/oder die Bewegung des Lanzettenhalters (15) nicht verhindert, hin und her bewegt werden kann, **dadurch gekennzeichnet, dass** sie ferner einen Auswurfschalter (26) umfasst, wobei der Auswurfschalter (26) zwischen einer Auswurfstellung, in der er eine Relativbewegung eines Auswurfstiftes (16) und des Lanzettenhalters (15) zum Auswerfen einer Lanzette gestattet, und einer Ladestellung, in der er keinen Einfluss auf eine gekoppelte Bewegung des Auswurfstiftes (16) und des Lanzettenhalters (15) hat, hin und her bewegt werden kann, wobei der Sicherheitsschalter (23) und der Auswurfschalter (26) auf einem gemeinsamen Schaltelement (22) angeordnet sind.

2. Lanzettenvorrichtung nach Anspruch 1, **dadurch gekennzeichnet, dass** der Auswurfschalter (26) in der Ladestellung die Bewegung des Auswurfstiftes (16), insbesondere zusammen mit einer Rückwärtsbewegung des Lanzettenhalters (15) ermöglicht.

3. Lanzettenvorrichtung nach Anspruch 1, **dadurch gekennzeichnet, dass** das Schaltelement (22) axial verschiebbar ist.

4. Lanzettenvorrichtung nach Anspruch 3, **dadurch gekennzeichnet, dass** die Lanzettenvorrichtung (1) eine Sicherheitsfeder (24) zum Schieben des Schaltelements (22) umfasst.

5. Lanzettenvorrichtung nach einem der Ansprüche 1-4, **dadurch gekennzeichnet, dass** die Vorderkappe (3) entfernbar ist und der Sicherheitsschalter (23) durch Entfernen der Vorderkappe (3) in die Sicherheitsstellung gebracht werden kann und/oder der Auswurfschalter (26) durch Entfernen der Vorderkappe (3) in die Auswurfstellung gebracht werden kann.

6. Lanzettenvorrichtung nach einem der Ansprüche 1-5, **dadurch gekennzeichnet, dass** der Sicherheitsschalter (23) durch die Vorderkappe (3) in der Passierstellung gehalten wird und/oder der Auswurfschalter (26) durch die Vorderkappe (3) in der Ladestellung gehalten wird, vorzugsweise gegen die Federkraft einer Sicherheitsfeder (24), solange die Vorderkappe (3) befestigt ist.

7. Lanzettenvorrichtung nach einem der Ansprüche 1-6, **dadurch gekennzeichnet, dass** die Lanzettenvorrichtung (1) ein Bedienmittel (9), vorzugsweise ein Mittel (9) zur Freigabe des Lanzettenhalters (15) aus der gespannten Position, umfasst und dass die Lanzettenvorrichtung (1) so konstruiert ist, dass der sich in der Sicherheitsstellung befindliche Sicherheitsschalter (23) die Bewegung des Lanzettenhalters (15) in die gespannte Position je nach Betätigung des Bedienmittels verhindert, insbesondere solange das Bedienmittel (9) nicht betätigt wird.

8. Lanzettenvorrichtung nach einem der Ansprüche 1-7, **dadurch gekennzeichnet, dass** die Lanzettenvorrichtung ein Außengehäuse (2) und ein Innengehäuse (20) umfasst, wobei das Innengehäuse einen ersten Auslegerbalken (25) zum Eingriff mit einer Anschlagfläche (28) eines Kupplungselements (18) des Lanzettenhalters (15) und/oder einen zweiten Auslegerbalken (27) zum Eingriff mit einer Anschlagfläche (29) des Auswurfstiftes (16) umfasst.

9. Lanzettenvorrichtung nach einem der Ansprüche 1-8, **dadurch gekennzeichnet, dass** die Lanzettenvorrichtung (1) ein Modusschalterelement (22) zum Umschalten zwischen einem Gebrauchszustand, worin die Betätigung eines Ladeelements (10) den Lanzettenhalter (15) spannt, und einem Auswechselzustand, in dem die Betätigung des Ladeelements (10) für das Auswerfen einer Lanzette (7), die sich im Lanzettenhalter (15) befindet, sorgt, umfasst.

10. Lanzettenvorrichtung nach Anspruch 9, **dadurch gekennzeichnet, dass** die Lanzettenvorrichtung (1) eine entfernbare Vorderkappe (3) umfasst und die Lanzettenvorrichtung (1) so konstruiert ist, dass das Modusschalterelement (22) in den Gebrauchszustand gebracht werden kann, wenn die Vorderkappe (3) befestigt ist, und das Modusschalterelement (22) in den Auswechselzustand gebracht werden kann, wenn die Vorderkappe (3) entfernt ist.

11. Lanzettenvorrichtung nach einem der Ansprüche 1-10, umfassend einen Lanzettenhalter (15), der axial zwischen einer gespannten Position und einer Stechposition hin und her gleiten kann, **dadurch gekennzeichnet, dass** die Lanzettenvorrichtung (1) ein Außengehäuse (2) und ein Innengehäuse (20) umfasst, wobei das Innengehäuse (20) zumindest zwei Führungsbahnen (30) umfasst, die zumindest zwei entsprechende Führungsrippen (31) auf dem Lanzettenhalter (15) führen.

## Revendications

1. Dispositif autopiqueur destiné à éjecter une lancette possédant un élément piqueur qui en fait saillie suivant un axe d'éjection, comprenant un embout avant (3) possédant une ouverture (5) contre laquelle un objet à piquer peut être placé, l'élément piqueur s'étendant à travers l'ouverture (5) lors de l'éjection de la lancette, un porte-lancette (15) apte à coulisser entre une position armée et une position de piqûre,
le dispositif autopiqueur (1) comprenant :
un commutateur de sécurité (23), ledit commutateur de sécurité (23) étant mobile entre une position de sécurité dans laquelle il empêche l'armement du porte-lancette (15) et une position de passage dans laquelle il n'empêche pas l'armement et/ou le mouvement du porte-lancette (15),
le dispositif autopiqueur (1) étant **caractérisé en ce qu'**il comprend en outre :
un commutateur d'éjection (26), ledit commutateur d'éjection (26) étant mobile entre une position d'éjection dans laquelle il permet un mouvement relatif entre une broche d'éjection (16) et le porte-lancette (15) en vue de l'éjection d'une lancette, et une position de chargement dans laquelle il n'a aucun effet sur un mouvement solidaire de la broche d'éjection (16) et du porte-lancette (15),
le commutateur de sécurité (23) et le commutateur d'éjection (26) étant montés sur un élément de commutation commun (22).

2. Dispositif autopiqueur selon la revendication 1, **caractérisé en ce que** ledit commutateur d'éjection (26) dans la position de chargement permet le mouvement de la broche d'éjection (16), plus particulièrement conjointement avec un mouvement de recul du porte-lancette (15).

3. Dispositif autopiqueur selon la revendication 1, **caractérisé en ce que** l'élément de commutation (22) est apte à coulisser axialement.

4. Dispositif autopiqueur selon la revendication 3, **caractérisé en ce qu'**il comprend un ressort de sécurité (24) destiné à pousser l'élément de commutation (22).

5. Dispositif autopiqueur selon l'une quelconque des revendications 1 à 4, **caractérisé en ce que** l'embout avant (3) est détachable et le commutateur de sécurité (23) est apte à être amené dans la position de sécurité par détachage de l'embout avant (3) et/ou le commutateur d'éjection (26) est apte à être amené dans la position d'éjection par détachage de l'embout avant (3).

6. Dispositif autopiqueur selon l'une quelconque des revendications 1 à 5, **caractérisé en ce que** le commutateur de sécurité (23) est maintenu dans la position de passage par l'embout avant (3) et/ou le commutateur d'éjection (26) est maintenu dans la position de chargement par l'embout avant (3), de préférence à l'encontre de la force de ressort exercée par un ressort de sécurité (24), tant que l'embout avant (3) est attaché.

7. Dispositif autopiqueur selon l'une quelconque des revendications 1 à 6, **caractérisé en ce qu'**il comprend un moyen d'actionnement (9), de préférence un moyen (9) destiné à libérer le porte-lancette (15) de la position armée, et le dispositif autopiqueur (1) est conçu de telle sorte que le commutateur de sécurité (23) occupant la position de sécurité empêche le mouvement du porte-lancette (15) vers la position armée en fonction de l'actionnement du moyen d'actionnement, plus particulièrement tant que le moyen d'actionnement (9) n'est pas actionné.

8. Dispositif autopiqueur selon l'une quelconque des revendications 1 à 7, **caractérisé en ce qu'**il comprend un boîtier extérieur (2) et un boîtier intérieur (20), le boîtier intérieur comprenant une première tige en porte-à-faux (25) destinée à venir au contact d'une face d'arrêt (28) d'un élément d'enclenchement (18) du porte-lancette (15) et/ou une deuxième tige en porte-à-faux (27) destinée à venir au contact d'une face d'arrêt (29) de la broche d'éjection (16).

9. Dispositif autopiqueur selon l'une quelconque des revendications 1 à 8, **caractérisé en ce qu'**il comprend un élément de commutation de mode (22) destiné à assurer la commutation entre un état d'utilisation dans lequel l'actionnement d'un élément de chargement (10) provoque l'armement du porte-lancette (15), et un état d'échange dans lequel l'actionnement de l'élément de chargement (10) provoque l'éjection d'une lancette (7) placée dans le porte-lancette (15).

10. Dispositif autopiqueur selon la revendication 9, **caractérisé en ce qu'**il comprend un embout avant (3) détachable et est conçu de telle sorte que l'élément de commutation de mode (22) est apte à être amené dans l'état d'utilisation lorsque l'embout avant (3) est attaché et l'élément de commutation de mode (22) est apte à être amené dans l'état d'échange lorsque l'embout avant (3) est détaché.

11. Dispositif autopiqueur selon l'une quelconque des revendications 1 à 10, comprenant un porte-lancette (15) apte à coulisser axialement entre une position armée et une position de piqûre,
le dispositif autopiqueur (1) étant **caractérisé en ce qu'**il comprend un boîtier extérieur (2) et un boîtier intérieur (20), le boîtier intérieur (20) comprenant au moins deux chemins de guidage (30) qui guident au moins deux nervures de guidage (31) correspondantes sur le porte-lancette (15).
